Europäisches Patentamt

European Patent Office

Office européen des brevets

(19)

(11) Veröffentlichungsnummer: **0 074 075**
**B1**

(12) **EUROPÄISCHE PATENTSCHRIFT**

(45) Veröffentlichungstag der Patentschrift:
**12.12.84**

(21) Anmeldenummer: **82108027.2**

(22) Anmeldetag: **01.09.82**

(51) Int. Cl.³: **C 07 C 1/20,** C 07 C 11/02 //
B01J29/04

(54) Verfahren zur Herstellung von Olefinen aus Methanol und/oder Dimethylether.

(30) Priorität: **09.09.81 DE 3135618**

(43) Veröffentlichungstag der Anmeldung:
**16.03.83 Patentblatt 83/11**

(45) Bekanntmachung des Hinweises auf die Patenterteilung:
**12.12.84 Patentblatt 84/50**

(84) Benannte Vertragsstaaten:
**BE DE FR GB IT NL**

(56) Entgegenhaltungen:
**DE - A - 2 827 385**
**US - A - 3 998 899**
**US - A - 4 035 430**
**US - A - 4 254 297**
**US - A - 4 268 420**

(73) Patentinhaber: **BASF Aktiengesellschaft,
Carl-Bosch-Strasse 38, D-6700 Ludwigshafen (DE)**

(72) Erfinder: **Hoelderich, Wolfgang, Dr., Mannheimer
Strasse 18 C, D-6710 Frankenthal (DE)**
Erfinder: **Mross, Wolf, Dieter Dr.,
Anselm-Feuerbach-Strasse 21, D-6710 Frankenthal (DE)**
Erfinder: **Schwarzmann, Matthias, Dr.,
Carl-Bosch-Strasse 54, D-6703 Limburgerhof (DE)**

BUNDESDRUCKEREI BERLIN

## Beschreibung

Es sind eine Reihe Verfahren zur Herstellung von niederen Olefinen aus Methanol und/oder Dimethylether bekannt.

In neuerer Zeit gewinnen Bemühungen, Methanol zur Herstellung von Olefinen zu verwenden, zunehmendes Interesse. Methanol kann man aus Kohle, durch Kohlevergasung und Herstellung von Synthesegas durch Konvertierung, mit Hilfe bewährter Technik herstellen. Gelingt es, Methanol in technisch einfacher Weise in niedere Olefine umzuwandeln, können die heute üblichen Weiterverarbeitungsverfahren auch bei der Verwendung von Kohle als Rohstoff beibehalten werden. In den vergangenen Jahren sind daher eine Reihe von Verfahren entwickelt worden, die die Herstellung von Olefinen aus Methanol und/oder Dimethylether zum Gegenstand haben. Ein solches Verfahren ist beispielsweise in der DE-OS 2 615 150 beschrieben. Als Katalysator wird dabei der Aluminosilikatzeolith ZMS-5 verwendet. Alle bisher bekannt gewordenen Verfahren haben jedoch den Nachteil, daß sie nicht wirtschaftlich sind entweder des geringen Methanolumsatzes wegen, oder wegen des geringen Olefinanteils bei einstufiger Fahrweise. Unter technisch gut realisierbaren Reaktionsbedingungen ergeben die bekannten Zeolithkatalysatoren vom Pentasil-Typ überwiegend flüssige Kohlenwasserstoffe.

Aus US-A-4 035 430 ist schon ein Verfahren bekannt, bei dem man in zwei Stufen aus Methanol über den Dimethylether durch Spaltung über zeolithischen Katalysatoren ein stark aromatenhaltiges Benzin erhält, hierbei werden die anfallenden Durene zur Erhöhung der Aromatenausbeute in die Spaltung zurückgeführt.

Es wurde nun gefunden, daß man die obengenannten Nachteile bei der Herstellung von niederen Olefinen aus Methanol und/oder Dimethylether durch katalytische Umsetzung bei Temperaturen von 300 bis 550°C in Gegenwart von Zeolithkatalysatoren vermeidet, wenn man die Umsetzung in zwei Stufen an Zeolithkatalysatoren ausführt, wobei nach der ersten Umsetzungsstufe $C_2-C_4$-Olefine und $C_1-C_4$-Paraffine bzw. nur das Ethylen und Propylen abgetrennt werden, die $C_5^+$-Kohlenwasserstoffe bzw. diese, die Butene und $C_1-C_4$-Paraffine in die zweite Umsetzungsstufe geführt und aus dem Umsetzungsprodukt der zweiten Stufe die Aromaten abgetrennt und die übrigen Reaktionsprodukte zurückgeführt werden.

Besonders geeignet für die erfindungsgemäße Durchführung des Verfahrens sind Borsilikatzeolithe.

Eine besondere Ausführungsform besteht darin, daß man das Umsetzungsprodukt der zweiten Stufe nach Abtrennung der Aromaten in die Aufarbeitungsstufe nach der ersten Umsetzung zurückführt, in der die enthaltenen $C_2-C_4$-Olefine mit den aus der ersten Umsetzungsstufe kommenden $C_2-C_4$-Olefinen abgetrennt werden und die verbleibenden Kohlenwasserstoffe in die zweite Umsetzungsstufe gelangen.

Der wesentliche Fortschritt des erfindungsgemäßen Verfahrens besteht darin, daß durch die Verfahrensführung nach Abtrennung des geringen Aromatenanteils nach der zweiten Verfahrensstufe alle übrigen Anteile zurückgeführt und zu den erwünschten $C_2-C_4$-Olefinen verarbeitet werden. Dieser Fortschritt in Richtung höherer $C_2-C_4$-Olefin-Ausbeuten wird insbesondere durch die Anwendung von Borosilikatzeolithen als Katalysatoren für die Umwandlung in beiden Stufen bewirkt. Die Borosilikatkatalysatoren drängen im Gegensatz zu anderen Katalysatoren für die Umwandlung von Methanol die Bildung von Aromaten zurück. Die an dieser Stelle entstehenden höheren Aliphate können in der zweiten Stufe weiter zu niederen Olefinen umgesetzt werden.

Es wurde weiterhin gefunden, daß man besonders vorteilhaft die katalytische Umsetzung des Methanols und/oder Dimethylethers in der ersten Reaktionsstufe bei Temperaturen von 350 bis 425°C ausführt, da man hierdurch den Aromatenanteil der $C_5^+$-Kohlenwasserstofffraktion zugunsten der Aliphate herabsetzt. Ein weiterer Vorteil dieser bevorzugten Ausführungsform des Verfahrens besteht darin, daß die von Temperatur und Belastung abhängigen Standzeiten der verwendeten Katalysatoren bei dem Temperaturbereich von 350 bis 425°C bei gleicher Belastung ein Optimum aufweisen.

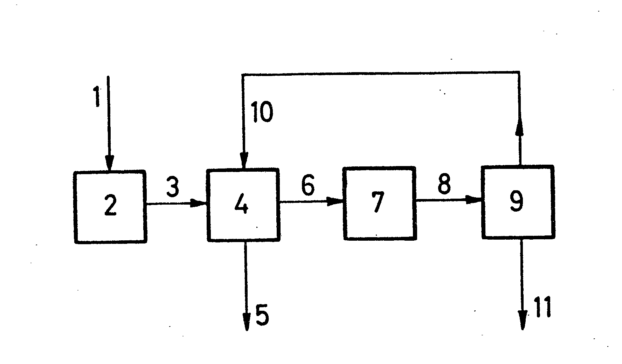

Das in der Zeichnung (Fig. 1) wiedergegebene Verfahrensschema verdeutlicht den grundlegenden Verfahrensablauf. Aus Leitung (1) gelangen die Ausgangsstoffe Methanol und/oder Dimethylether in den ersten Reaktor (2) des Zweistufenprozesses in dem die Umwandlung von Methanol und/oder Dimethylether in ein Kohlenwasserstoffgemisch an einem Zeolithkatalysator durchgeführt wird. Die Reaktionsprodukte, die $C_2-C_4$-Olefine, $C_1-C_4$-Paraffine, flüssige Kohlenwasserstoffe ($C_5^+$) mit einem hohen Anteil an nichtaromatischen Verbindungen und Wasser enthalten, gelangen durch Leitung (3) in die erste Aufarbeitungsstufe (4).

Als Katalysatoren für diese erste Stufe eignen sich insbesondere Borsilikatzeolithe vom Pentasil-Typ. Die Reaktionsprodukte aus der ersten Stufe werden in (4) in bekannter Weise getrennt. Nach Abtrennung der $C_2-C_4$-Olefine, $C_1-C_4$-Paraffine und des Wassers über Produktleitung (5), durch die sie zur weiteren Trennung geführt werden, gelangen über Leitung (6) die $C_5^+$-Kohlenwasserstoffe in den zweiten Reaktor (7), in dem sie erneut an einem Zeolithkatalysator umgesetzt werden. Auch hierfür werden vorteilhaft Borsilikatzeolithe als Katalysatoren verwendet. Diese Reaktion kann mit Verdünnungsmittel, vorzugsweise Wasser, im Verhältnis 1:2 bis 2:1 als auch ohne Verdünnungsmittel durchgeführt werden. In diesem Fall kann man auf die Abtrennung des Wassers in (4) verzichten.

Die im Reaktor (7) erhaltenen Kohlenwasserstoffe gelangen durch Leitung (8) in die zweite Aufarbeitungsstufe (9) in der sie von den Aromatenanteilen in bekannter Weise befreit werden und über Leitung (10) den Reaktionsprodukten aus der ersten Stufe in (4) zugeführt werden. Die rückgeführten Anteile enthalten $C_2-C_4$-Olefine, $C_1-C_4$-Paraffine und $C_5^+$-Aliphate. Eine alternative Ausführungsform besteht darin, daß man in der Aufarbeitungsstufe (4) nur Ethylen und Propylen abtrennt und die $C_5^+$-Kohlenwasserstoffe zusammen mit $C_1-C_4$-Paraffinen, Butenen und Wasser durch Leitung (6) in den zweiten Reaktor (7) führt. Die in (9) abgetrennten Aromatenanteile werden über die Leitung (11) abgezogen.

Im folgenden wird die Herstellung des bevorzugt angewendeten Katalysators beschrieben:

Ein Borosilikatzeolith wird durch hydrothermale Synthese aus 66,1 g $SiO_2$ (Aerosil 200), 30,85 g $H_3BO_3$ in 881 g einer wäßrigen 1,3 Propandiamin-Lösung (Mischung 50 : 50) gefällt bzw. kristallisiert, bei 170°C 24 h getrocknet und bei 500°C 24 h calciniert. Dieser Borosilikatzeolith setzt sich zusammen aus 91,2% $SiO_2$, 4,74% $B_2O_3$, 0,015% $Na_2O$. Nach Verstrangung des Zeoliths mit Boehmit im Verhältnis 60 : 40 erhält man den in den nachfolgend beschriebenen Versuchen verwendeten Katalysator.

In Tabelle 1 sind die Ergebnisse der ersten Stufe des hier beschriebenen Prozesses wiedergegeben:

An dem hergestellten Katalysator wird Rohmethanol bei verschiedenen Temperaturen und Belastungen im isothermen Reaktor, das ist ein Wendelreaktor aus V2A, Innendurchmesser 0,6 cm und Länge 90 cm, zu Kohlenwasserstoffen umgewandelt. Reaktionsbedingungen und Ausbeuten — bezogen auf eingesetztes $CH_2$ — werden in Tabelle 1 wiedergegeben.

Tabelle 1

| | Versuch 1 | 2 | 3 | 4 | 5 |
|---|---|---|---|---|---|
| Temperatur | 400°C | 375°C | 400°C | 450°C | 500°C |
| WHSV | 3,9 h⁻¹ | 7,8 h⁻¹ | 7,8 h⁻¹ | 7,8 h⁻¹ | 7,8 h⁻¹ |
| $CH_4$ | 1,3% | 0,6% | 1,0% | 2,4% | 3,7% |
| $C_2H_4$ | 5,3% | 5,4% | 5,5% | 7,1% | 9,5% |
| $C_2H_6$ | 0,2% | 0,1% | 0,1% | 0,2% | 0,3% |
| $C_3H_6$ | 15,9% | 13,0% | 18,1% | 31,3% | 36,9% |
| $C_3H_8$ | 2,5% | 2,4% | 2,2% | 1,4% | 1,2% |
| $C_4H_8$ | 16,1% | 15,1% | 16,4% | 18,0% | 16,8% |
| $C_4H_{10}$ | 7,8% | 2,0% | 8,2% | 3,9% | 2,0% |
| $C_5$ | 50,8% | 60,7% | 48,2% | 35,5% | 29,0% |
| davon BTX-Aromaten | 7,2% | 6,7% | 6,3% | 5,7% | 8,4% |
| g $CH_3OH$/g Katalysator*) | 350 g | 312 g | 413 g | 375 g | 218 g |

*) Laufzeit bis zur ersten Regenerierung.

Ein repräsentativer $C_5^+$-Schnitt, wie er aus der ersten Stufe des Prozesses nach Abtrennen der $C_2-C_4$-Olefine erhalten wird, besteht zu 75,7 Gew.-% aus Aliphaten und zu 24,3 Gew.-% aus Aromaten. Ein solches Gemisch wird am Borosilikatkatalysator mit und ohne Wasserzugabe zu niederen Olefinen im $C_2-C_4$-Bereich umgesetzt. Die Versuchsergebnisse sind in Tabelle 2 zusammengefaßt:

Tabelle 2

| | Beispiel (ohne $H_2O$-Zugabe) | (mit $H_2O$-Zugabe) |
|---|---|---|
| Temperatur | 500° C | 500° C |
| WHSV | 4,7 h$^{-1}$ | 4,8$^{-1}$ |
| $C_5^+ : H_2O$ | — | 50% : 50% |
| Umsatz der $C_5^+$-Aliphate | 39,7% | 49% |
| Selektivität gasförmige Produkte $C_1-C_4$ | 56,3% | 79,4% |
| Selektivität Aromaten | 43,7% | 20,6% |
| Selektivität der Einzelkomponenten im Gas | | |
| $CH_4$ | 0,6% | 0,3% |
| $C_2H_4$ | 8,9% | 14,0% |
| $C_2H_6$ | 0,9% | 0,5% |
| $C_3H_6$ | 21,7% | 37,9% |
| $C_3H_8$ | 5,3% | 3,3% |
| $C_4H_8$ | 14,2% | 15,5% |
| $C_4H_{10}$ | 4,5% | 7,6% |

Aus den Versuchen der Tabelle 2 erkennt man, daß durch Umsetzung der $C_5^+$-Aliphate in einer zweiten Reaktionsstufe die Gesamtausbeute an niederen Olefinen gesteigert werden kann und durch die $H_2O$-Zugabe eine Erhöhung der Selektivität des Katalysators zu gasförmigen $C_1-C_4$-Produkten erfolgt.

**Patentansprüche**

1. Verfahren zur Herstellung von niederen Olefinen aus Methanol und/oder Dimethylether durch katalytische Umsetzung bei Temperaturen von 300 bis 550° C in Gegenwart von Zeolithkatalysatoren, dadurch gekennzeichnet, daß man die Umsetzung in zwei Stufen an Zeolithkatalysatoren ausführt, wobei nach der ersten Umsetzungsstufe $C_2-C_4$-Olefine und $C_1-C_4$-Paraffine bzw. nur das Ethylen und Propylen abgetrennt, die $C_5^+$-Kohlenwasserstoffe bzw. diese, die Butene und $C_1-C_4$-Paraffine in die zweite Umsetzungsstufe geführt und aus dem Umsetzungsprodukt der zweiten Stufe die Aromaten abgetrennt und die übrigen Reaktionsprodukte zurückgeführt werden.

2. Verfahren nach Anspruch 1, dadurch gekennzeichnet, daß man als Katalysator einen Borosilikatzeolithen verwendet.

3. Verfahren nach Anspruch 1 und 2, dadurch gekennzeichnet, daß man das Umsetzungsprodukt der zweiten Stufe nach Abtrennung der Aromaten in die Aufarbeitungsstufe nach der ersten Umsetzung zurückführt, in der die enthaltenen $C_2-C_4$-Olefine mit den aus der ersten Umsetzungsstufe kommenden $C_2-C_4$-Olefinen abgetrennt werden und die verbleibenden Kohlenwasserstoffe in die zweite Umsetzungsstufe gelangen.

# 0 074 075

## Claims

1. A process for the preparation of lower olefins from methanol and/or dimethyl ether by catalytic conversion at from 300 to 550° C in the presence of zeolite catalysts, wherein the reactin is carried out over zeolite catalysts in two stages, $C_2-C_4$-olefins and $C_1-C_4$-paraffins, or only the ethylene and propylene, being removed after the first reaction stage, the $C_5^+$ hydrocarbons, or these, the butenes and the $C_1-C_4$-paraffins, being passed to the second reaction stage, the aromatics being removed from the reaction product of the second stage and the remaining reaction product being recycled.

2. A process as claimed in claim 1, wherein a borosilicate zeolite is used as the catalyst.

3. A process as claimed in claims 1 and 2, wherein, after removal of the aromatics, the reaction product from the second stage is recycled to the working-up stage which follows the first reaction, and the $C_2-C_4$-olefins contained in the product are removed in this working-up stage, together with the $C_2-C_4$-olefins from the first reaction stage, and the remaining hydrocarbons are passed to the second reaction stage.

## Revendication

1. Procédé de préparation d'oléfines inférieures à partir de l'alcool méthylique et(ou) de l'éther diméthylique par une réaction catalysée par des zéolithes, entre 300 et 550° C, caractérisé en ce que la réaction en présence de catalyseurs zéolithiques est réalisée en deux stades et du mélange réactionnel provenant du premier stade, on extrait soit les oléfines en $C_2$ à $C_4$ et les paraffines en $C_1$ à $C_4$, soit l'éthylène et le propylène, tandis que les hydrocarbures en $C_5$ et plus et éventuellement les butènes et les paraffines en $C_1$ à $C_4$ sont amenés dans le deuxième stade de réaction, les substances aromatiques formées dans le deuxième stade étant ensuite séparées et les autres produits réacionnels recyclés dans le procédé.

2. Procédé suivant la revendication 1, caractérisé en ce que le catalyseur est une zéolithe boro-silica-tée.

3. Procédé suivant la revendication 1 ou la revendication 2, caractérisé en ce que le produit réactionnel obtenu dans le deuxième stade est recyclé, après la séparation des substances aromatiques, dans l'opération d'extraction du mélange réactionnel provenant du premier stade, dans laquelle les oléfines en $C_2$ à $C_4$ formées dans le premier stade et celles formées dans le deuxième stade sont séparées, les autres hydrocarbures étant amenés dans le deuxième stade opératoire.